# FASCICULE DE BREVET EUROPEEN

(11) **EP 2 005 910 B1**
(45) Date de publication et mention de la délivrance du brevet: **20.02.2019**
(21) Numéro de dépôt: 08290587.8
(22) Date de dépôt: 19.06.2008
(51) Int. Cl.: A61B 17/80

(54) **Kit de fixation à usage médical ou chirurgical**
Fixierungskit für den medizinischen oder chirurgischen Einsatz
Attachment kit for medical or surgical use

(30) Priorité: 21.06.2007 FR 0704449
(43) Date de publication de la demande: 24.12.2008
(73) Titulaire: Newdeal, 69006 Lyon (FR)
(72) Inventeur: Rouyer, Guillaume Francois, 01600 Massieux (FR); Fourcault, Eric Stéphane, 69130 Ecully (FR); Gauneau, Bertrand Xavier Francois, 69570 Dardilly (FR)
(74) Mandataire: Weber, Jean-François

(56) Documents cités:
- DE-A1-102005 004 841
- US-A- 6 139 550
- US-A1- 2004 215 195
- US-A1- 2005 070 904
- US-B2- 6 890 335

## Description

La présente invention se rapporte au domaine technique des dispositifs destinés à être solidarisés à un support, en vue par exemple de consolider ou de réparer ledit support, ou encore pour doter ce support d'un élément complémentaire fonctionnel constitué par le dispositif.

Plus précisément, la présente invention se rapporte au domaine médical, et en particulier au domaine des dispositifs de chirurgie, notamment osseuse, utilisables par exemple en chirurgie orthopédique, en traumatologie, ou en chirurgie reconstructrice.

La présente invention concerne ainsi un kit de fixation à usage médical ou chirurgical comprenant une pièce d'assemblage destinée à être accouplée à un support biologique et pourvue d'au moins un premier logement traversant ménagé en son sein, ledit kit comprenant un premier et un deuxième organe de fixation comportant chacun une tête et une tige destinée à être ancrée dans ledit support.

Il est déjà connu d'assurer une ostéosynthèse à l'aide d'un kit comprenant d'une part une plaque d'assemblage pourvue de lumières et d'autre part des vis de fixation destinées à être reçues dans lesdites lumières pour assurer la solidarisation de la plaque avec le support osseux à traiter.

Afin d'obtenir une bonne efficacité thérapeutique, il est important d'assurer une fixation ferme de la vis relativement à la plaque, pour éviter une mobilité entre la vis et la plaque qui nuirait à la rigidité de l'assemblage osseux réalisé et pourrait même conduire à un arrachement des vis. A cette fin, il est classique de mettre en oeuvre des kits comprenant d'une part des vis à tête tronconique, c'est-à-dire dont la tête présente une face inférieure de forme tronconique, et d'autre part des plaques dont chaque lumière définit une portée tronconique d'appui pour la tête de vis correspondante.

Ainsi, chaque tête de vis vient en appui, par sa face inférieure tronconique, contre une portée tronconique complémentaire, réalisant ainsi un appui surfacique qui est de nature à limiter la mobilité de la tête de vis relativement à la plaque, une fois la vis en place.

Ces kits classiques ne sont toutefois pas adaptés à certaines situations nécessitant de positionner une ou plusieurs vis selon une orientation (angulation) particulière relativement à la plaque, cette orientation étant par exemple fonction de la configuration osseuse spécifique concernée et/ou de la nature et de la géométrie des lésions à traiter.

Dans ce cas, la mise en oeuvre d'un kit classique semblable à ceux qui ont été décrits dans ce qui précède n'est pas appropriée, car elle ne permet pas au praticien de choisir l'orientation de la vis par rapport à la plaque. Dans ces kits classiques, l'incidence des vis est en effet fixe et prédéfinie par l'orientation de la portée tronconique qui guide la tête de vis.

Afin de remédier à ce problème, il a été proposé des kits dans lesquels les lumières de la plaque définissent des portées sphériques, tandis que les têtes de vis associées ont elles-mêmes une forme sphérique. Cela permet d'obtenir un guidage de type rotule entre la tête de vis et la portée contre laquelle la tête de vis vient en appui. Un tel guidage sphérique permet de faire varier l'orientation de la vis relativement à la plaque dans un angle solide, de sorte que le praticien est en mesure de choisir l'angulation de la vis. Le document US-A-6,139,550 décrit un système plaque/vis mettant en oeuvre un guidage de ce genre entre la tête de vis et la portée, avec un élément de verrouillage de la vis.

Ces kits d'ostéosynthèse à vis orientables connus souffrent toutefois d'un niveau de solidarisation entre la tête et la plaque moins bon que celui obtenu avec les kits classique à portées et vis tronconiques décrits dans ce qui précède.

Cette relative faiblesse de solidarisation provient bien entendu de la nature même de la liaison entre la tête de vis sphérique et la portée sphérique correspondante, qui autorise précisément une certaine liberté angulaire de la vis.

Cette faiblesse de solidarisation entre la tête de vis sphérique et la portée sphérique correspondante est d'autant plus regrettable que dans un grand nombre de situations opératoires, la faculté de mobilité angulaire des vis n'est utilisée que pour une petite minorité d'entre elles, les autres vis n'ayant pas forcément besoin d'une orientation particulière et étant généralement fixées selon une orientation normale à la plaque. En d'autres termes, les kits à vis orientables connus proposent une fonction d'orientation angulaire disponible pour chaque lumière ménagée dans la plaque, alors que cette fonction n'est en pratique pas toujours nécessaire, ou alors n'est utile qu'au niveau de quelques lumières seulement (qui ne sont toutefois pas connues à l'avance, ni en nombre, ni en localisation). Bien évidemment, pour les lumières au niveau desquelles une possibilité de variation angulaire n'est pas nécessaire, il aurait été préférable de disposer d'un guidage tronconique dans la mesure où ce dernier permet une meilleure stabilité de la solidarisation entre la tête et la plaque.

Toutefois, dans nombre de situations opératoires, les lumières au niveau desquelles une faculté d'orientation angulaire est nécessaire ne sont généralement pas connues à l'avance, ni en nombre, ni en localisation sur la plaque. De façon réciproque, les lumières au niveau desquelles il sera nécessaire d'assurer une excellente solidarisation de la tête de vis avec la plaque ne sont pas non plus connues à l'avance.

Cela oblige les chirurgiens à disposer de plusieurs types de kits (à vis orientables et à vis non orientables), ce qui complique la gestion hospitalière et augmente donc les coûts afférents à cette gestion. Surtout, cela conduit les chirurgiens à faire des compromis en matière de fixation, en privilégiant au cas par cas une fonction (par exemple la mobilité angulaire) au détriment d'une autre (par exemple la stabilité de la solidarisation), ce qui ne permet souvent pas d'offrir au patient un effet thérapeutique optimal.

On connaît par ailleurs du document US-2005/070904A1 des plaques destinées à coopérer avec soit des vis de blocage soit des vis de compression.

L'objet de l'invention vise en conséquence à porter remède aux différents inconvénients énumérés précédemment et à proposer un nouveau kit de fixation à usage médical ou chirurgical simple, bon marché et dont la conception permet de réaliser un assemblage présentant un excellent compromis entre rigidité et stabilité d'une part et adaptabilité aux contraintes anatomiques d'autre part.

Un autre objet de l'invention vise à proposer un nouveau kit de fixation à usage médical ou chirurgical qui permette d'obtenir un assemblage particulièrement stable et qui offre une excellente résistance à l'arrachement. Un autre objet de l'invention vise à proposer un nouveau kit de fixation à usage médical ou chirurgical de construction particulièrement simple et fiable.

Un autre objet de l'invention vise à proposer un nouveau kit de fixation à usage médical ou chirurgical permettant d'obtenir un assemblage démontable dont la stabilité et la résistance à l'arrachement sont optimisées. Les objets assignés à l'invention sont atteints à l'aide d'un kit de fixation à usage médical ou chirurgical selon la revendication 1.

D'autres objets et avantages de l'invention apparaîtront plus en détails à la lecture de la description qui suit, et à l'aide des dessins annexés fournis à titre purement illustratif et non limitatif, dans lesquels :
- La figure 1 illustre, selon une vue générale en perspective, un exemple de kit de fixation conforme à l'invention, ledit kit étant destiné à être implanté chirurgicalement pour réaliser une ostéosynthèse.
- La figure 2 est une vue agrandie en coupe selon la ligne A-A de la figure 1.
- La figure 3 illustre, selon une vue partielle agrandie en coupe, la coopération du premier logement de la pièce d'assemblage avec d'une part une vis osseuse à tête fraisée constituant le premier organe de fixation et d'autre part un plot fileté plat constituant le premier organe de blocage.

- La figure 4 illustre, selon une vue partielle agrandie en coupe, la coopération du premier logement de la pièce d'assemblage avec d'une part une vis osseuse à tête sphérique constituant le deuxième organe de fixation et d'autre part un plot fileté bombé constituant le second organe de blocage.
- La figure 5 illustre, selon une vue en perspective, le premier organe de fixation seul, lequel est en l'espèce constitué par une vis osseuse à tête fraisée.
- La figure 6 illustre, selon une vue en perspective, le deuxième organe de fixation seul, lequel est en l'espèce constitué par une vis osseuse à tête sphérique.
- La figure 7 illustre, selon une vue en perspective, un plot fileté plat destiné à constituer un premier organe de blocage pour la vis illustrée à la figure 5.
- La figure 8 illustre, selon une vue en coupe, le plot fileté plat de la figure 7.
- La figure 9 illustre, selon une vue en perspective, un plot fileté bombé constituant le deuxième organe de blocage pour la vis illustré à la figure 6.
- La figure 10 illustre, selon une vue en coupe, le plot fileté bombé de la figure 9.

L'invention concerne un kit de fixation 1 à usage médical ou chirurgical. Le kit de fixation 1 conforme à l'invention est de préférence destiné à être implanté dans le corps humain, par voie chirurgicale. Ainsi, la variante du kit 1 conforme à l'invention illustrée aux figures est destinée à être utilisée en chirurgie orthopédique ou en traumatologie pour réaliser une ostéosynthèse, en vue par exemple de réduire une fracture osseuse.

Le kit 1 conforme à l'invention comprend une pièce d'assemblage 2 destinée à être accouplée à un support biologique (non illustré). Dans l'exemple illustré aux figures, la pièce d'assemblage 2 est un implant chirurgical, et plus précisément une plaque chirurgicale d'ostéosynthèse.

Dans cet exemple, le support biologique auquel la pièce d'assemblage 2 est destinée à être accouplée est donc de nature osseuse. Dans ce cas, la plaque formant pièce d'assemblage 2 est destinée à assurer une liaison mécanique entre les différents fragments de l'os fracturé, comme cela est d'ailleurs bien connu en tant que tel.

L'invention n'est toutefois pas limitée à une pièce d'assemblage 2 constituée par une plaque d'ostéosynthèse, et il est par exemple tout à fait envisageable que la pièce d'assemblage 2 soit constituée par une plaque d'arthrodèse (pour l'immobilisation d'une articulation) ou soit constituée par un implant cotyloïdien, ou encore par un implant de toute autre nature, voire même par un ancillaire chirurgical.

L'invention n'est pas non plus limitée à un support biologique de nature osseuse et peut concerner d'autres supports biologiques, y compris des tissus relativement mous, ou du moins présentant une rigidité inférieure à celle des os.

De préférence, la pièce d'assemblage 2 est d'un seul tenant, c'est-à-dire qu'elle présente un caractère unitaire monobloc. La pièce d'assemblage 2 peut être réalisée en tout matériau implantable présentant des caractéristiques mécaniques compatibles avec l'utilisation recherchée. Ainsi, dans le cas où la pièce d'assemblage 2 constitue, comme cela est illustré aux figures, une plaque d'ostéosynthèse, cette dernière peut être par exemple réalisée en un matériau métallique tel que l'acier inoxydable ou le titane.

Conformément à l'invention, la pièce d'assemblage 2 est pourvue d'au moins un premier logement traversant 3 ménagé en son sein. En d'autres termes, le premier logement 3 forme une lumière qui traverse la pièce d'assemblage 2 et débouche de part et d'autre de cette dernière.

Dans l'exemple illustré aux figures, le premier logement 3 est ainsi constitué par un orifice pratiqué de part en part de la pièce d'assemblage 2, dans toute l'épaisseur de cette dernière, entre sa face supérieure 2A et sa face inférieure 2B opposée. Ladite face inférieure 2B est destinée à être positionnée en regard du support biologique à consolider.

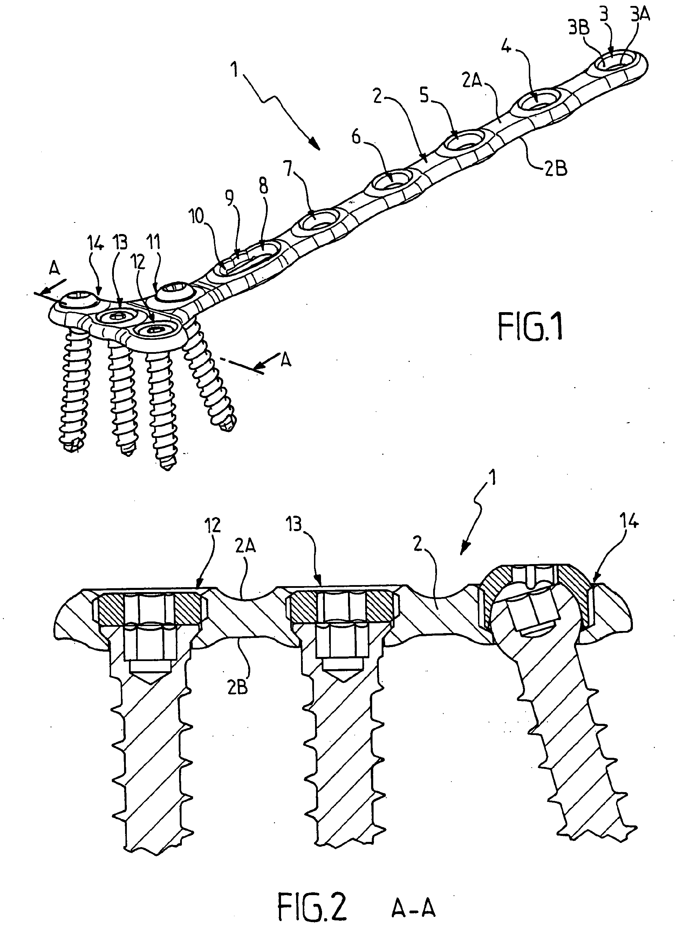

Avantageusement et comme illustré à la figure 1, la pièce d'assemblage 2 comprend, outre le premier logement traversant 3, au moins un deuxième logement traversant 4, de conception sensiblement similaire, et de préférence identique, à celle du premier logement 3. Dans l'exemple illustré à la figure 1, la pièce d'assemblage 2 comprend même une pluralité de logements traversants 3, 4, 5, 6, 7, 11, 12, 13, 14 identiques les uns aux autres et répartis sur la surface de la pièce d'assemblage 2. Dans ce même exemple, la pièce d'assemblage 2 est pourvue, en plus des neuf logements traversants identiques 3, 4, 5, 6, 7, 11, 12, 13, 14, de trois logements 8, 9, 10 communiquant entre eux de la façon décrite dans la demande de brevet français publiée sous le numéro FR-2 886 535 A1.

Il est bien évidemment également envisageable que la pièce d'assemblage 2 comporte d'autres logements de conceptions différentes, sans pour autant que l'on sorte du cadre de l'invention.

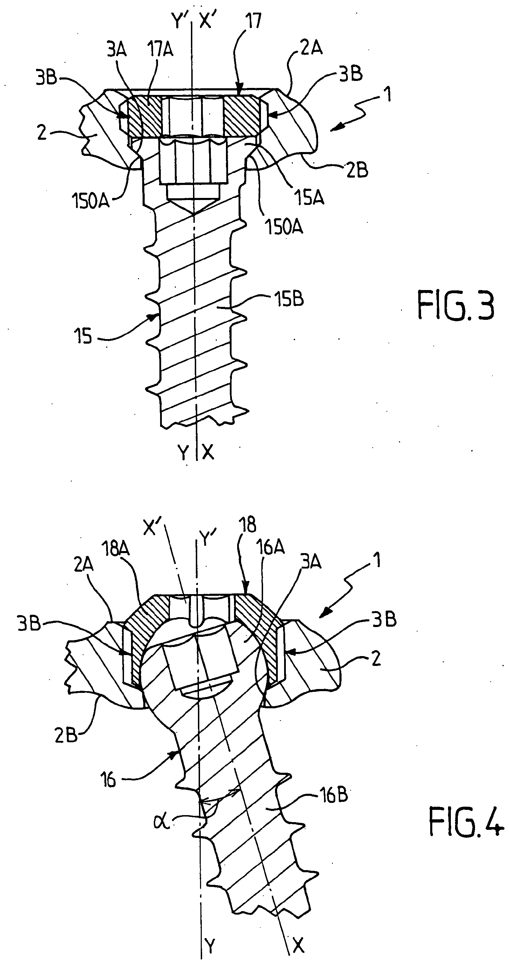

Selon l'invention, le kit 1 comprend un premier et un deuxième organe de fixation 15, 16, le premier logement 3 étant conçu pour coopérer indifféremment avec l'un ou l'autre desdits premier et deuxième organes de fixation 15, 16, au choix de l'utilisateur. De préférence, le premier logement 3 est ainsi conçu pour coopérer individuellement avec les premier et deuxième organes de fixation 15, 16, lesquels sont illustrés respectivement aux figures 3 et 4.

Les premier et deuxième organes de fixation 15, 16 comportent chacun, comme cela est connu en soi, une tête 15A, 16A et une tige 15B, 16B s'étendant longitudinalement à partir de la tête 15A, 16A selon un axe d'extension principal X-X'. Les tiges 15A, 16A sont bien entendu destinées à être ancrées dans le support biologique.

De préférence, lesdits premier et deuxième organes de fixation 15, 16 sont respectivement constitués, tel que cela est illustré aux figures 3 et 4, par une première et une deuxième vis, lesdites vis étant en l'espèce des vis osseuses. Dans ce cas, le sommet de la tête 15A, 16A de chaque organe de fixation est pourvu d'une empreinte de serrage, du genre empreinte à six pans, destinée à recevoir une clé de serrage correspondante en vue d'effectuer un vissage de la tige 15A, 16A dans le support biologique.

Bien évidemment, l'invention n'est pas limitée à la mise en oeuvre d'organes de fixation 15, 16 constitués par des vis et il est tout à fait envisageable que lesdits organes de fixation 15, 16 soient constitués par tout autre moyen alternatif bien connu de l'homme du métier, et par exemple par des clous ou des chevilles.

Conformément à l'invention, le premier logement 3 est conformé pour réaliser un siège 3A contre lequel la tête 15A, 16A de l'organe de fixation 15, 16 choisi est destiné à venir en appui, tel que cela est notamment illustré aux figures 2 à 4.

En d'autres termes, dans le mode de réalisation illustré aux figures, le premier logement 3 est conçu pour accueillir individuellement la tête 15A, 16A de l'un ou l'autre des premier et deuxième organes de fixation 15, 16, au choix de l'utilisateur.

De préférence, le siège 3A est réalisé par une restriction de la section de l'orifice constituant le logement 3, cette restriction de section formant une portée contre laquelle la tête 15A, 16A de la vis, qui est de diamètre supérieur à celui de la tige 15B, 16B qui la prolonge, vient en appui (cf. figures 2 à 4). De préférence, le siège 3A est réalisé vers le fond de l'orifice constituant le logement 3, c'est-à-dire vers la face inférieure 2B, de sorte que le logement 3 est ainsi en mesure de contenir sensiblement l'ensemble, ou du moins une fraction majoritaire, de la tête 15A, 16A qu'il accueille en son sein. Cela permet un assemblage à profil mince, qui est de ce fait atraumatique.

Conformément à une caractéristique importante de l'invention, le siège 3A et la tête 15A, 16A de chaque organe de fixation 15, 16 sont tous trois conformés les uns relativement aux autres pour que :
- d'une part la coopération du siège 3A et de la tête 15A du premier organe de fixation 15 immobilise sensiblement ce dernier dans une orientation prédéterminée par rapport à la pièce d'assemblage 2,
- et pour que d'autre part la coopération du siège 3A et de la tête 16A du deuxième organe de fixation 16 permette une variation de l'orientation de ce dernier par rapport à la pièce d'assemblage 2.

En d'autres termes, le kit 1 est conçu pour permettre la mise en oeuvre de deux configurations alternatives (au choix de l'utilisateur) au niveau du premier logement 3, chacune de ces configurations permettant d'obtenir une fonction spécifique.

Ainsi, dans la première configuration (illustrée à la figure 3), le premier logement 3 coopère avec le premier organe de fixation 15, c'est-à-dire que le premier logement 3 accueille individuellement la tête 15A du premier organe de fixation 15, de telle sorte que cette dernière vient en appui contre le siège 3A. Conformément à l'invention, la forme du siège 3A et la forme de la tête 15A sont conçues l'une par rapport à l'autre pour guider et maintenir sensiblement l'organe de fixation 15 dans une orientation prédéterminée et fixe par rapport à la pièce d'assemblage 2.

Cela signifie que lorsque le premier logement 3 coopère avec le premier organe de fixation 15, c'est-à-dire lorsque la tête 15A du premier organe de fixation 15 vient en appui contre le siège 3A du logement 3, la position angulaire du premier organe de fixation 15 est fixée de manière stable et définitive et ne peut sensiblement pas être modifiée par l'utilisateur.

Dans la seconde configuration (illustrée à la figure 4), le premier logement 3 coopère cette fois avec le deuxième organe de fixation 16, c'est-à-dire que le premier logement 3 accueille individuellement la tête 16A du second organe de fixation 16, de telle sorte que cette dernière vient en appui contre le siège 3A. La forme du siège 3A et la forme de la tête 16A sont conçues l'une par rapport à l'autre pour permettre une variation de l'orientation angulaire du deuxième organe de fixation 16 par rapport à la pièce d'assemblage 2. Avantageusement, dans cette deuxième configuration, le siège 3A assure un guidage de la tête 16A qui autorise cette dernière à se déplacer en rotation selon les trois axes de l'espace, c'est-à-dire que l'appui de la pièce 16A sur le siège 3A produit une liaison mécanique de type rotule entre ladite tête 16A et la pièce d'assemblage 2. Grâce à cette mesure technique, la tige 16B qui s'étend à partir de la tête 16A peut être déplacée manuellement par l'utilisateur dans un angle solide jusqu'à atteindre une position angulaire qui convient au praticien.

Ainsi, l'invention repose sur la mise en oeuvre d'un seul et unique siège 3A contre lequel peut venir en appui soit une tête 15A dont la conformation permet d'immobiliser sensiblement l'organe de fixation 15 correspondant dans une orientation prédéterminée, soit une tête 16A permettant à l'organe de fixation 16 correspondant une certaine liberté angulaire.

En d'autres termes, le principe général de l'invention repose sur la possibilité pour le praticien de choisir, pour un seul et même logement 3 ménagé dans la pièce d'assemblage 2 :
- soit un organe de fixation 15 privilégiant une solidarisation ferme et stable de la tête 15A de l'organe de fixation 15 et de la pièce d'assemblage 2, avec toutefois une absence de possibilité de choisir une orientation angulaire de l'organe de fixation par rapport à la pièce d'assemblage 2,
- soit un assemblage dont la tenue mécanique est *a priori* moindre, mais qui autorise une possibilité de choisir, dans un angle solide donné, l'orientation de l'organe de fixation 16 par rapport à la pièce d'assemblage 2.

Grâce à ces caractéristiques, le praticien est en mesure, en fonction de la situation anatomique, de choisir au cas par cas l'organe de fixation qui convient le mieux, en privilégiant soit la stabilité à l'interface plaque/vis, soit la mise en oeuvre d'un positionnement précis de la vis par rapport à la plaque et surtout à l'os dans lequel la vis pénètre.

L'invention est particulièrement avantageuse lorsque la pièce d'assemblage 2 comprend une pluralité de logements 4, 5, 6, 7, 8, 9, 10, 11, 12, 13, 14 similaires au premier logement 3, chaque logement 4, 5, 6, 7, 8, 9, 10, 11, 12, 13, 14 étant associé à deux organes de fixation respectivement similaires aux premier et deuxième organes de fixation 15, 16. De cette façon, le praticien est en mesure de choisir logement par logement, en fonction de la situation anatomique, quel est l'organe de fixation le plus approprié, ce qui lui permet d'obtenir en définitive un excellent compromis global entre rigidité et stabilité de fixation d'une part et polyvalence et adaptabilité à la situation anatomique d'autre part.

Ainsi, dans une version particulièrement avantageuse de l'invention, le kit 1 comprend au moins :
- non seulement un premier logement traversant 3 ainsi que des premier et deuxième organes de fixation 15, 16 associés, comme décrit dans ce qui précède,
- mais également d'une part au moins un deuxième logement traversant 4, de conception sensiblement similaire à celle du premier logement 3, ledit deuxième logement 4 étant de préférence identique au premier logement 3, et d'autre part au moins un troisième organe de fixation de conception sensiblement similaire à celle du premier organe de fixation 15, et de préférence identique à celle dudit premier organe de fixation 15, ainsi qu'un quatrième organe de fixation de conception sensiblement similaire à celle du deuxième organe de fixation 16, et de préférence identique à celle dudit deuxième organe de fixation 16, lesdits troisième et quatrième organes de fixation étant conçus pour coopérer avec le deuxième logement 4 de la même façon que lesdits premier et deuxième organes de fixation 15, 16 coopèrent avec le premier logement 3.

Bien évidemment, il existe une multitude de possibilités à la portée de l'homme du métier pour réaliser la double fonction mécanique visée par l'invention.

Selon l'exemple illustré aux figures, le siège 3A comprend ainsi une portée de forme sensiblement tronconique contre laquelle la tête 15A, 16A de l'organe de fixation 15, 16 choisi est destinée à venir en appui. Tel que cela est illustré aux figures, la portée tronconique formant le siège 3A présente un axe de symétrie Y-Y', qui est de préférence perpendiculaire au plan d'extension principal de la pièce d'assemblage 2. Bien évidemment, il est tout à fait envisageable de prévoir un logement 3 s'étendant en oblique, avec dans ce cas un axe de symétrie Y-Y' s'étendant selon une incidence oblique par rapport au plan d'extension principal de la pièce d'assemblage 2.

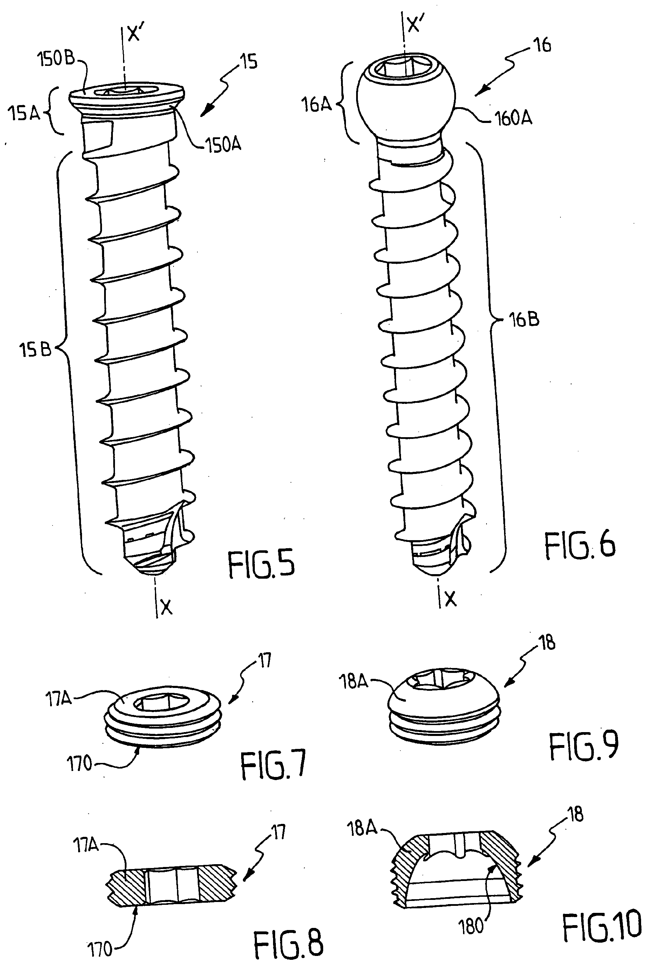

Avantageusement, la tête 15A du premier organe de fixation 15 comprend une face inférieure 150A destinée à venir en appui contre le siège 3A, ladite face inférieure présentant une forme sensiblement tronconique, et de préférence une forme de collerette tronconique tel qu'illustré à la figure 5. De préférence, la forme tronconique de la face inférieure 150A de la tête 15A est complémentaire de la forme tronconique du siège 3A, c'est-à-dire de la forme tronconique de la portée formant le siège 3A. Cela permet, comme illustré aux figures 2 et 3, de réaliser un appui plan de la face inférieure 150A contre le siège 3A. Cet appui plan selon une interface tronconique permet d'immobiliser la vis dans une orientation donnée correspondant à l'axe de symétrie Y-Y' du siège 3A, de sorte que l'axe X-X' d'extension de la tige 15B et l'axe Y-Y' sont confondus. Ainsi, ce contact troncone/troncone permet une solidarisation particulièrement stable et durable de l'organe de fixation 15 avec la pièce d'assemblage 2.

Avantageusement, la tête 16A du deuxième organe de fixation 16 comprend elle aussi une face inférieure 160A destinée à venir en appui contre le siège 3A, ladite face inférieure 160A étant en forme de portion de sphère, comme illustré à la figure 6. Ainsi, la tête sphérique 16A du deuxième organe de fixation 16 vient en appui contre la portée tronconique du siège 3A selon une zone d'appui sensiblement linéique (cf. figure 4), un tel appui linéique autorisant un débattement angulaire du deuxième organe de fixation 16 par rapport à la pièce d'assemblage 2, c'est à dire une variation, dans un angle solide, de l'angle α entre l'axe X-X' d'extension de la tige 16B et l'axe Y-Y' attaché au siège 3A. En d'autres termes, la tête 16A est logée de manière orientable dans le logement 3.

Avantageusement, le kit 1 conforme à l'invention comprend en outre au moins un organe de blocage 17, 18 conçu pour être fixé dans le premier logement 3 de façon à venir en appui contre la tête 15A, 16A de l'organe de fixation 15, 16 choisi. En d'autres termes, le premier logement 3 est avantageusement conçu pour coopérer individuellement avec l'organe de blocage 17, 18, de telle sorte que ce dernier exerce un effort de blocage sur la tête 15A, 16A de l'organe de fixation 15, 16 choisi, ledit effort permettant ainsi de réaliser un encastrement mécanique de la tête 15A, 16A relativement à la pièce d'assemblage 2.

A cette fin, l'organe de blocage 17, 18 est fixé dans le premier logement 3 de façon que la tête 15A, 16A de l'organe de fixation 15, 16 choisi soit interposée entre le siège 3A d'une part et l'organe de blocage 17, 18 d'autre part, ladite tête 15A, 16A étant ainsi comprimée entre lesdits sièges 3A et organe de blocage 17, 18.

La mise en oeuvre d'un organe de blocage 17, 18 permet de réaliser un scellement de la tête 15A, 16A de l'organe de fixation 15, 16 concerné, scellement qui contribue à l'obtention d'une excellente stabilité mécanique de l'assemblage ainsi réalisé. Une telle stabilité est bien évidemment tout à fait bénéfique d'un point de vue thérapeutique, puisqu'elle évite notamment une mobilité de l'organe de fixation 15, 16 relativement à la pièce d'assemblage 2, mobilité qui pourrait conduire à l'arrachement de l'organe de fixation 15, 16 sous l'effet des mouvements du patient.

La mise en oeuvre de l'organe de blocage 17, 18 permet ainsi, en combinaison avec les autres caractéristiques décrites précédemment, d'obtenir un kit qui tout en étant particulièrement simple, bon marché et polyvalent, présente une excellente stabilité mécanique quelle que soit la façon dont il est mis en oeuvre.

Avantageusement, le premier logement 3 comprend une portion taraudée 3B, l'organe de blocage 17, 18 comprenant quant à lui un plot fileté 17A, 18A destiné à être vissé dans la portion taraudée 3B.

Plus précisément, le logement 3A comprend avantageusement un orifice cylindrique d'axe de symétrie X-X' qui s'étend à partir de la face supérieure 2A de la pièce d'assemblage 2 jusqu'au siège 3A, lequel est situé à proximité de la face inférieure 2B. La paroi intérieure de cet orifice cylindrique est taraudée, c'est-à-dire qu'elle est pourvue d'un filetage en creux destiné à coopérer avec un filetage externe dont est pourvu le plot fileté 17A, 18A sur sa paroi latérale, ledit plot présentant également une symétrie de révolution comme illustré aux figures. Lorsque le plot 17A, 18A est vissé dans le logement 3A, son axe de symétrie est confondu avec l'axe de symétrie X-X' du logement 3A.

Il est tout à fait envisageable d'utiliser un unique plot fileté dont la conception permet qu'il coopère soit avec le premier organe de fixation 15, si l'utilisateur choisit la première configuration, soit avec le deuxième organe de fixation 16, si l'utilisateur choisit la deuxième configuration.

Toutefois, afin d'optimiser l'effet mécanique de blocage recherché, il est préférable que le kit 1 conforme à l'invention comprenne un premier et un deuxième organe de blocage 17, 18 présentant chacun une face de blocage 170, 180 destinée à venir en appui contre la tête 15A, 16A lorsque l'organe de blocage 17, 18 est fixé (en l'occurrence par vissage) dans le logement 3, la face de blocage 170 du premier organe de blocage 17 présentant une forme sensiblement complémentaire de celle de la tête 15A du premier organe de fixation 15, tandis que la face de blocage 180 du deuxième organe de blocage 18 présente une forme sensiblement complémentaire de celle de la tête 16A du deuxième organe de fixation 16. Ainsi, dans l'exemple de réalisation illustré aux figures, la tête 15A du premier organe de fixation 15 présente une face supérieure sensiblement plate, perpendiculaire à l'axe d'extension X-X' de la vis. Le premier organe de blocage 17 destiné à coopérer spécifiquement avec le premier organe de fixation 15 comprend alors une face de blocage 170, qui correspond à sa face inférieure, elle aussi de forme sensiblement plate, de manière à favoriser un contact plan entre la face supérieure 150B de la tête 15A et la face de blocage 170 du plot fileté 17A formant le premier organe de blocage 17.

Dans l'exemple illustré aux figures, la tête 16A du second organe de fixation 16 présente quant à elle une forme sensiblement sphérique, et plus précisément une forme de sphère tronquée vers sa partie supérieure. Dans ce cas, la face de blocage 180 du deuxième organe de blocage 18 destiné à coopérer spécifiquement avec le second organe de fixation 16 présente une forme incurvée sphérique, c'est-à-dire qu'elle correspond à une coupelle de contour sphérique ménagée à l'intérieur du deuxième organe de blocage 18, comme illustré à la figure 10. Cette forme spécifique de la face de blocage 180 permet une parfaite congruence, et donc un appui surfacique, de la face de blocage 180 sur la tête de vis 16A. Un tel appui surfacique est bien évidemment de nature à favoriser une excellente tenue mécanique et donc un effet de blocage optimisé.

En définitive, il est particulièrement avantageux de prévoir, pour chaque logement 3, 4, 5, 6, 7, 8, 9, 10, 11, 12, 13, 14, deux organes de fixation sensiblement identiques respectivement au premier et au deuxième organe de fixation 15, 16 décrits dans ce qui précède, ainsi que deux organes de blocage respectivement sensiblement identiques aux premier et deuxième organe de blocage 17, 18 décrits dans ce qui précède.

Cela permet au praticien de choisir, pour chaque logement, l'organe de fixation le plus adapté et l'organe de blocage correspondant.

## Revendications

1. Kit de fixation (1) destiné à être implanté chirurgicalement et comprenant une pièce d'assemblage (2) destinée à être accouplée à un support biologique et pourvue d'au moins un premier logement traversant (3) ménagé en son sein, ledit kit (1) comprenant un premier et un deuxième organe de fixation (15, 16) comportant chacun une tête (15A, 16A) et une tige (15B, 16B) destinée à être ancrée dans ledit support, ledit premier logement (3) étant conçu pour coopérer indifféremment avec l'un ou l'autre desdits premier et deuxième organe de fixation (15, 16) au choix de l'utilisateur, ledit logement (3) étant conformé pour réaliser un siège (3A) comprenant une portée de forme sensiblement tronconique contre laquelle la tête (15A, 16A) de l'organe de fixation (15, 16) choisi est destinée à venir en appui, le siège (3A) et la tête (15A, 16A) de chaque organe de fixation (15, 16) étant conformés les uns relativement aux autres pour que d'une part la coopération du siège (3A) et de la tête (15A) du premier organe de fixation (15) immobilise sensiblement ce dernier dans une orientation prédéterminée par rapport à la pièce d'assemblage (2), la tête (15A) du premier organe de fixation (15) comprenant une face inférieure (150A) de forme sensiblement tronconique destinée à venir en appui contre la portée de forme sensiblement tronconique, et pour que d'autre part la coopération du siège (3A) et de la tête (16A) du deuxième organe de fixation (16) permette une variation de l'orientation de ce dernier par rapport à la pièce d'assemblage (2), ledit kit comprenant en outre au moins un organe de blocage (17, 18) conçu pour être fixé dans le premier logement (3) de façon à venir en appui contre la tête (15A, 16A) de l'organe de fixation (15, 16) choisi.

2. Kit (1) selon la revendication 1 **caractérisé en ce que** l'organe de blocage (17, 18) est fixé dans le premier logement (3) de façon que la tête (15A, 16A) de l'organe de fixation (15, 16) choisi soit interposée entre le siège (3A) et l'organe de blocage (17, 18).

3. Kit (1) selon la revendication 2 **caractérisé en ce que** le premier logement (3) comprend une portion taraudée (3B), l'organe de blocage (17, 18) comprenant quant à lui un plot fileté (17A, 18A) destiné à être vissé dans la portion taraudée (3B).

4. Kit (1) selon l'une des revendications 1 à 3 **caractérisé en ce qu'**il comprend un premier et un deuxième organe de blocage (17, 18) présentant chacun une face de blocage (170, 180) destinée à venir en appui contre la tête (15A, 16A) lorsque l'organe de blocage (15, 16) est fixé dans le logement (3), la face de blocage (170) du premier organe de blocage (17) présentant une forme sensiblement complémentaire de celle de la tête (15A) du premier organe de fixation (15), tandis que la face de blocage (180) du deuxième organe de blocage (18) présente une forme sensiblement complémentaire de celle de la tête (16A) du deuxième organe de fixation (16).

5. Kit (1) selon l'une des revendications 1 à 4 **caractérisé en ce que** la tête (16A) du deuxième organe de fixation (16) comprend une face inférieure (160A) destinée à venir en appui contre le siège (3A), ladite face inférieure (160A) étant en forme de portion de sphère.

6. Kit (1) selon l'une des revendications 1 à 5 **caractérisé en ce qu'**il comprend d'une part au moins un deuxième logement traversant (4), de conception sensiblement similaire à celle du premier logement (3), et d'autre part au moins un troisième organe de fixation, de conception sensiblement similaire à celle du premier organe de fixation (15) ainsi qu'un quatrième organe de fixation, de conception sensiblement similaire à celle du deuxième organe de fixation (16), lesdits troisième et quatrième organes de fixation étant conçus pour coopérer avec le deuxième logement (4) de la même façon que lesdits premier et deuxième organes de fixation (15, 16) coopèrent avec le premier logement (3).

7. Kit (1) selon l'une des revendications 1 à 6 **caractérisé en ce que** lesdits premier et deuxième organes de fixation (15, 16) sont respectivement constitués par une première et une deuxième vis.

8. Kit (1) selon l'une des revendications 1 à 7 **caractérisé en ce que** ledit support biologique est de nature osseuse, tandis que la pièce d'assemblage (2) est un implant ou un ancillaire chirurgical.

9. Kit (1) selon la revendication 8 **caractérisé en ce que** la pièce d'assemblage (2) est une plaque d'ostéosynthèse ou d'arthrodèse.

## Patentansprüche

1. Fixierungskit (1), das dazu bestimmt ist, chirurgisch implantiert zu werden und aufweisend ein Verbindungsstück (2) aufweist, das dazu bestimmt ist, mit einem biologischen Träger verbunden zu werden und mit mindestens einer ersten durchgehenden Aufnahme (3) versehen ist, die darin ausgebildet ist, wobei das Kit (1) ein erstes und ein zweites Befestigungsorgan (15, 16) aufweist, die jeweils einen Kopf (15A, 16A) und eine Stange (15B, 16B) aufweisen, die dazu bestimmt ist, in dem Träger verankert zu werden, wobei die erste Aufnahme (3) ausgelegt ist, um mit dem einen oder dem anderen des ersten und zweiten Befestigungsorgans (15, 16) nach Wahl des Benutzers gleichermaßen zusammenzuwirken, wobei die Aufnahme (3) ausgebildet ist, um einen Sitz (3A) zu bilden, der eine im Wesentlichen kegelstumpfförmige Ausladung aufweist, gegen die der Kopf (15A, 16A) des ausgewählten Befestigungsorgans (15, 16) bestimmt ist, in Anlage zu kommen, wobei der Sitz (3A) und der Kopf (15A, 16A) von jedem Befestigungsorgan (15, 16) relativ zueinander derart ausgebildet sind, dass einerseits das Zusammenwirken des Sitzes (3A) und des Kopfes (15A) des ersten Befestigungsorgans (15) dieses Letztere in einer vorbestimmten Ausrichtung in Bezug auf das Verbindungsstück (2) im Wesentlichen immobilisiert, wobei der Kopf (15A) des ersten Befestigungsorgans (15) eine untere Fläche (150A) von im Wesentlichen kegelstumpfförmiger Form aufweist, die dazu bestimmt ist, gegen die im Wesentlichen kegelstumpfförmige Ausladung in Anlage zu kommen, und dass andererseits das Zusammenwirken des Sitzes (3A) und der Kopfes (16A) des zweiten Befestigungsorgans (16) eine Variation der Ausrichtung dieses Letzteren in Bezug auf das Verbindungsstück (2) ermöglicht, wobei das Kit ferner mindestens ein Blockierorgan (17, 18) aufweist, das ausgelegt ist, um in der ersten Aufnahme (3) derart befestigt zu werden, um gegen den Kopf (15A, 16A) des ausgewählten Befestigungsorgans (15, 16) in Anlage zu kommen.

2. Kit (1) nach Anspruch 1, **dadurch gekennzeichnet, dass** das Blockierorgan (17, 18) in der ersten Aufnahme (3) derart befestigt ist, dass der Kopf (15A, 16A) des ausgewählten Befestigungsorgans (15, 16) zwischen dem Sitz (3A) und dem Blockierorgan (17, 18) angeordnet ist.

3. Kit (1) nach Anspruch 2, **dadurch gekennzeichnet, dass** die erste Aufnahme (3) einen mit einem Innengewinde versehenen Abschnitt (3B) aufweist, wobei das Blockierorgan (17, 18) seinerseits einen Gewindestift (17A, 18A) aufweist, der dazu bestimmt ist, in den mit einem Innengewinde versehenen Abschnitt (3B) geschraubt zu werden.

4. Kit (1) nach einem der Ansprüche 1 bis 3, **dadurch gekennzeichnet, dass** es ein erstes und ein zweites Blockierorgan (17, 18) aufweist, die jeweils eine Blockierfläche (170, 180) aufweisen, die dazu bestimmt ist, gegen den Kopf (15A, 16A) in Anlage zu kommen, wenn das Blockierorgan (15, 16) in der Aufnahme (3) befestigt ist, wobei die Blockierfläche (170) des ersten Blockierorgans (17) ein Form aufweist, die im Wesentlichen komplementär zu jener des Kopfes (15A) des ersten Befestigungsorgans (15) ist, während die Blockierfläche (180) des zweiten Blockierorgans (18) eine Form aufweist, die im Wesentlichen komplementär zu jener des Kopfes (16A) des zweiten Befestigungsorgans (16) ist.

5. Kit (1) nach einem der Ansprüche 1 bis 4, **dadurch gekennzeichnet, dass** der Kopf (16A) des zweiten Befestigungsorgans (16) eine untere Fläche (160A) aufweist, die dazu bestimmt ist, gegen den Sitz (3A) in Anlage zu kommen, wobei die untere Fläche (160A) in Form eines Kugelabschnitts ist.

6. Kit (1) nach einem der Ansprüche 1 bis 5, **dadurch gekennzeichnet, dass** es einerseits mindestens eine zweite durchgehende Aufnahme (4), die im Wesentlichen gleichartig wie die erste Aufnahme (3) ausgebildet ist, und andererseits mindestens ein drittes Befestigungsorgan, das im Wesentlichen gleichartig wie das erste Befestigungsorgan (15) ausgebildet ist, sowie ein viertes Befestigungsorgan aufweist, das im Wesentlichen gleichartig wie das zweite Befestigungsorgan (16) ausgebildet ist, wobei das dritte und vierte Befestigungsorgan ausgelegt sind, um mit der zweiten Aufnahme (4) auf die gleiche Weise zusammenzuwirken, wie das erste und zweite Befestigungsorgan (15, 16) mit der ersten Aufnahme (3) zusammenwirken.

7. Kit (1) nach einem der Ansprüche 1 bis 6, **dadurch gekennzeichnet, dass** das erste und zweite Befestigungsorgan (15, 16) jeweils aus einer ersten und einer zweiten Schraube gebildet sind.

8. Kit (1) nach einem der Ansprüche 1 bis 7, **dadurch gekennzeichnet, dass** der biologische Träger knöcherner Art ist, während das Verbindungsstück (2) ein Implantat oder ein chirurgisches Hilfsinstrument ist.

9. Kit (1) nach Anspruch 8 **dadurch gekennzeichnet, dass** das Verbindungsstück (2) eine Osteosynthese- oder Arthrodeseplatte ist.

## Claims

1. Fixing kit (1) intended to be surgically implanted and comprising an assembly piece (2) intended to be coupled to a biological support and provided with at least one first through housing (3) provided in it, said kit (1) comprising first and second fixing members (15, 16) each comprising a head (15A, 16A), and a rod (15B, 16B) intended to be anchored in said support, said first housing (3) being designed to cooperate indifferently with either one or other of said first and second fixing members (15, 16) at the choice of the user, said housing (3) being conformed to produce a seat (3A) comprising a substantially frustoconically shaped span against which the head (15A, 16A) of the chosen fixing member (15, 16) is intended to come into abutment, the seat (3A) and the head (15A, 16A) of each fixing member (15, 16) being conformed in relation to one another so that firstly the cooperation of the seat (3A) and head (15A) of the first fixing member (15) substantially immobilises the latter in a predetermined orientation with respect to the assembly piece (2), the head (15A) of the first fixing member (15) comprising a bottom face (150A) with a substantially frustoconical shape intended to come into abutment against the substantially frustoconically shaped span, and so that secondly the cooperation of the seat (3A) and head (16A) of the second fixing member (16) allows a variation in the orientation of the latter with respect to the assembly piece (2), said kit further comprising at least one locking member (17, 18) designed so as to be fixed in the first housing (3) so as to come into abutment against the head (15A, 16A) of the chosen fixing member (15, 16).

2. Kit (1) according to claim 1, **characterised in that** the locking member (17, 18) is fixed in the first housing (3) so that the head (15A, 16A) of the chosen fixing member (15, 16) is interposed between the seat (3A) and the locking member (17, 18).

3. Kit (1) according to claim 2, **characterised in that** the first housing (3) comprises an internally threaded portion (3B), the locking member (17, 18) for its part comprising a threaded stud (17A, 18A) intended to be screwed into the internally threaded portion (3B).

4. Kit (1) according to any of claims 1 to 3, **characterised in that** it comprises first and second locking members (17, 18) each having a locking face (170, 180) intended to come into abutment against the head (15A, 16A) when the locking member (15, 16) is fixed in the housing (3), the locking face (170) of the first locking member (17) having a shape substantially complementary to that of the head (15A) of the first fixing member (15), while the locking face (180) of the second locking member (18) has a shape substantially complementary to that of the head (16A) of the second fixing member (16).

5. Kit (1) according to any of claims 1 to 4, **characterised in that** the head (16A) of the second fixing member (16) comprises a bottom face (160A) intended to come into abutment against the seat (3A), said bottom face (160A) being in the form of a portion of a sphere.

6. Kit (1) according to any of claims 1 to 5, **characterised in that** it comprises firstly at least one second through housing (4), of a design substantially similar to that of the first housing (3), and secondly at least one third fixing member, of a design substantially similar to that of the first fixing member (15) as well as a fourth fixing member, of a design substantially similar to that of the second fixing member (16), said third and fourth fixing members being designed to cooperate with the second housing (4) in the same way as said first and second fixing members (15, 16) cooperate with the first housing (3).

7. Kit (1) according to any of claims 1 to 6, **characterised in that** said first and second fixing members (15, 16) consist respectively of a first and second screw.

8. Kit (1) according to any of claims 1 to 7, **characterised in that** said biological support is bone in nature, while the assembly piece (2) is an implant or a surgical ancillary.

9. Kit (1) according to claim 8, **characterised in that** the assembly piece (2) is an osteosynthesis or arthrodesis plate.
